# EUROPEAN PATENT APPLICATION

(11) **EP 3 834 775 A1**
(43) Date of publication of application: **16.06.2021**
(21) Application number: 19848023.8
(22) Date of filing: 06.08.2019
(51) Int. Cl.: A61F 2/16

(54) **INTRAOCULAR LENS INSERTION IMPLEMENT**

(30) Priority: 07.08.2018 JP 2018148879
(71) Applicant: Santen Pharmaceutical Co., Ltd., Kita-ku Osaka-shi Osaka 530-8552 (JP)
(72) Inventor: MATSUMOTO, Kazuma, Osaka-shi, Osaka 533-8651 (JP)
(74) Representative: Müller-Boré & Partner Patentanwälte PartG mbB
(86) International application number: PCT/JP2019/030897
(87) International publication number: WO 2020/032023

(57) **Abstract**

In order to appropriately insert a plate-type intraocular lens, which is made of a flexible material, into an eye without causing a breakage of the plate-type intraocular lens, an insertion implement (1) of the present invention is configured such that a plunger (5) includes a lens contact member (54) which is made of an elastically deformable material and which is configured to be brought into contact with an intraocular lens (7) and the lens contact member (54) includes a protrusion (54a) which is configured to restrict elongational deformation of the intraocular lens (7) in a direction opposite to a direction in which the intraocular lens (7) is pushed out by the plunger (5).

## Description

### Technical Field

The present invention relates to an intraocular lens insertion implement.

### Background Art

As conventional techniques, intraocular lens insertion implements are known which are configured such that intraocular lenses held by lens holders are pushed out by plungers and is then released (for example, Patent Literatures 1 through 3). An intraocular lens insertion implement disclosed in each of Patent Literatures 1 through 3 includes a nozzle part from which, by being pushed by a plunger, an intraocular lens held by a lens holder is released while the intraocular lens is being folded in a recessed shape. This nozzle part is configured such that an inner diameter thereof gradually becomes smaller as the nozzle part extends forward.

### Citation List

### [Patent Literature]

[Patent Literature 1]
   Japanese Patent No. 5415452
[Patent Literature 2]
   Japanese Patent No. 3779819
[Patent Literature 3]
   Japanese Patent No. 5236638

### Summary of Invention

### Technical Problem

The intraocular lens to be mounted on the conventional intraocular lens insertion implement disclosed in each of Patent Literatures 1 through 3 includes a lens part, a front support part, and a rear support part. The front support part and the rear support part are formed so as to curvedly extend from respective side surfaces of the lens part. In this manner, each of the front support part and the rear support part has the shape of a loop.

Commercially available intraocular lenses include plate-type intraocular lenses each having a substantially rectangular outer shape, in addition to intraocular lenses each having a front support part and a rear support part each of which has the shape of a loop as described above. The plate-type intraocular lenses are often made of flexible materials, compared to the intraocular lenses each having the front support part and the rear support part each of which has the shape of a loop. Therefore, in a case where a plate-type intraocular lens is inserted into an eye with use of the intraocular lens insertion implement disclosed in each of Patent Literatures 1 through 3, the plate-type intraocular lens may be broken.

An object of an aspect of the present invention is to realize an intraocular lens insertion implement which makes it possible to appropriately insert a plate-type intraocular lens, which is made of a flexible material, into an eye without causing a breakage of the plate-type intraocular lens.

### Solution to Problem

In order to attain the above object, an intraocular lens insertion implement in accordance with an aspect of the present invention is an intraocular lens insertion implement to which a lens holder configured to hold an intraocular lens is attachable, including: a plunger which is configured to be brought into contact with the intraocular lens held by the lens holder and which is configured to cause the intraocular lens to be pushed out; and a nozzle part from which the intraocular lens that has been pushed out by the plunger is released outside while the intraocular lens is being folded, the plunger including a front end part which is configured to be brought into contact with the intraocular lens and which is made of an elastically deformable material, the front end part including a stopper which is configured to restrict elongational deformation of the intraocular lens in a direction opposite to a direction in which the intraocular lens is pushed out by the plunger.

In order to attain the above object, an intraocular lens insertion implement in accordance with an aspect of the present invention is an intraocular lens insertion implement to which a lens holder configured to hold an intraocular lens is attachable, including: a plunger which is configured to be brought into contact with the intraocular lens held by the lens holder and which is configured to cause the intraocular lens to be pushed out; and a nozzle part from which the intraocular lens that has been pushed out by the plunger is released outside while the intraocular lens is being folded, the nozzle part including: a plate-like rib part which is provided so as to be located in the middle of the nozzle part and which extends in a direction in which the intraocular lens is pushed out by the plunger; and a rotational mechanism which is configured to rotate the plate-like rib part in the direction in which the intraocular lens is pushed out by the plunger, by (i) contact of a lens surface of the intraocular lens with the plate-like rib part, (ii) contact of the plunger with the plate-like rib part, or (iii) contact of both of the lens surface of the intraocular lens and the plunger with the plate-like rib part.

In order to attain the above object, an intraocular lens insertion implement in accordance with an aspect of the present invention is an intraocular lens insertion implement to which a lens holder configured to hold an intraocular lens is attachable, including: a plunger which is configured to be brought into contact with the intraocular lens held by the lens holder and which is configured to cause the intraocular lens to be pushed out; and a nozzle part from which the intraocular lens that has been pushed out by the plunger is released outside while the intraocular lens is being folded, the nozzle part including: a protruding part which is provided so as to be located in the middle of the nozzle part and which protrudes toward a bore of the nozzle part and extends in a direction in which the intraocular lens is pushed out by the plunger; and a rotational mechanism which is configured to rotate the protruding part in the direction in which the intraocular lens is pushed out by the plunger, by (i) contact of a lens surface of the intraocular lens with the protruding part, (ii) contact of the plunger with the protruding part, or (iii) contact of both of the lens surface of the intraocular lens and the plunger with the protruding part.

In order to attain the above object, an intraocular lens insertion implement in accordance with an aspect of the present invention is an intraocular lens insertion implement to which a lens holder configured to hold an intraocular lens is attachable, including: a plunger which is configured to be brought into contact with the intraocular lens held by the lens holder and which is configured to cause the intraocular lens to be pushed out; and a nozzle part from which the intraocular lens that has been pushed out by the plunger is released outside while the intraocular lens is being folded, the nozzle part having a tapered inner wall surface which is configured such that a distance between wall surfaces that face each other becomes shorter as the wall surfaces extend in a direction in which the intraocular lens is pushed out by the plunger, the tapered inner wall surface being provided with a ridge part which extends in the direction in which the intraocular lens is pushed out by the plunger and which is configured to restrict rotational movement of the intraocular lens along the tapered inner wall surface.

In order to attain the above object, an intraocular lens insertion implement in accordance with an aspect of the present invention is an intraocular lens insertion implement which includes a body having a shape of a tube and to which a lens holder configured to hold an intraocular lens is attachable on an end surface side of the body, including: a plunger which is configured to (i) be inserted into the body so as to be capable of entering and leaving the body, (ii) be brought into contact with the intraocular lens held by the lens holder, and (iii) cause the intraocular lens to be pushed out; and a nozzle part from which the intraocular lens that has been pushed out by the plunger is released while the intraocular lens is being folded, the body being provided with a centering member which is configured to center the plunger.

### Advantageous Effects of Invention

According to an aspect of the present invention, it is possible to realize an intraocular lens insertion implement which makes it possible to appropriately insert a plate-type intraocular lens, which is made of a flexible material, into an eye without causing a breakage of the plate-type intraocular lens.

### Brief Description of Drawings

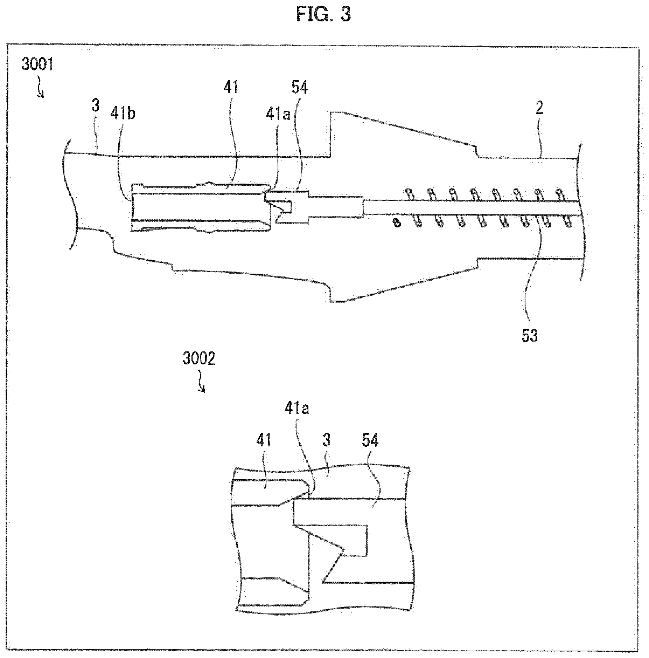

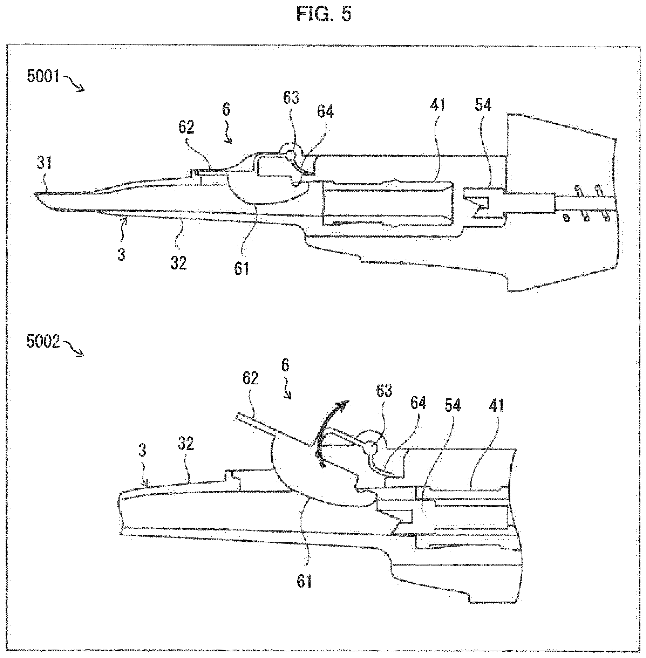

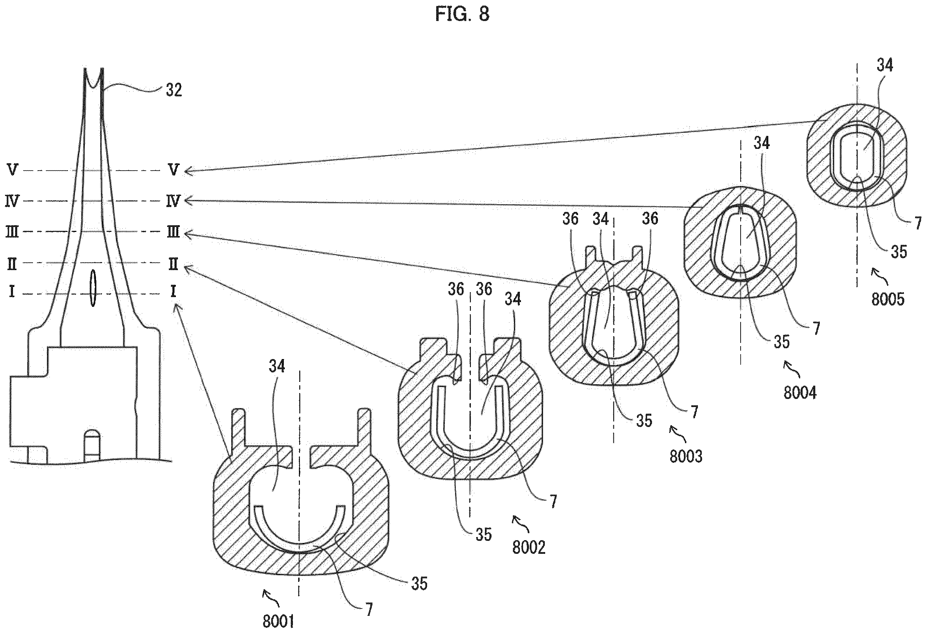

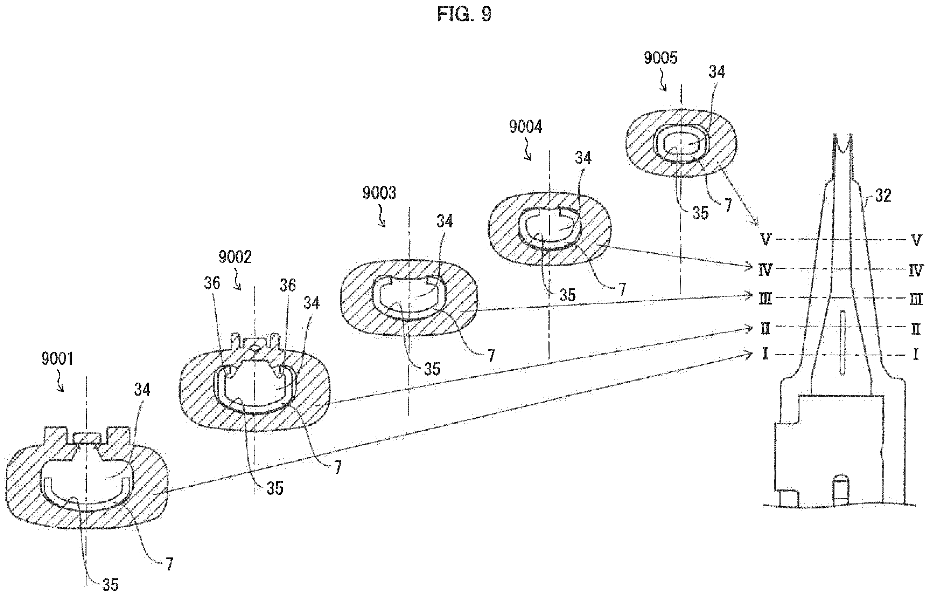

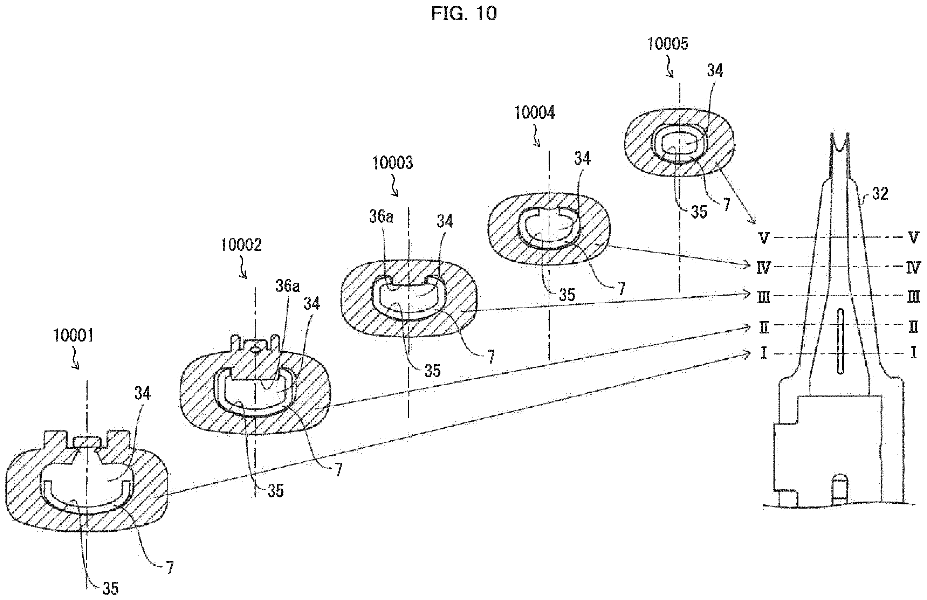

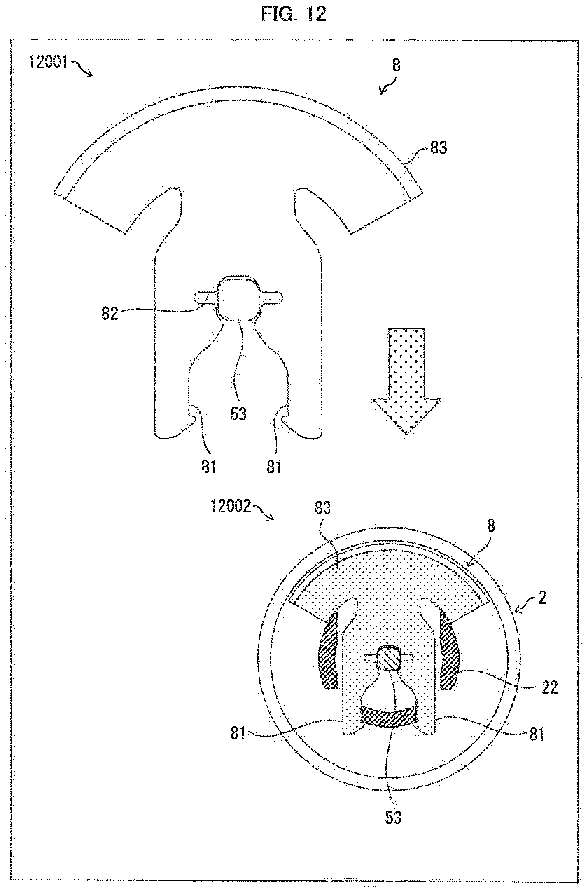

Fig. 1 is a perspective view illustrating an entire configuration of an intraocular lens insertion implement in accordance with an embodiment of the present invention.
Fig. 2 is a top view illustrating a configuration of an intraocular lens to be mounted on an intraocular lens insertion implement in accordance with an embodiment of the present invention.
3001 of Fig. 3 is a cross-sectional view illustrating a state where a lens contact member of a plunger of an intraocular lens insertion implement in accordance with an embodiment of the present invention is brought to an opening on a rear side of a housing part. 3002 of Fig. 3 is an enlarged view of the state illustrated in 3001.

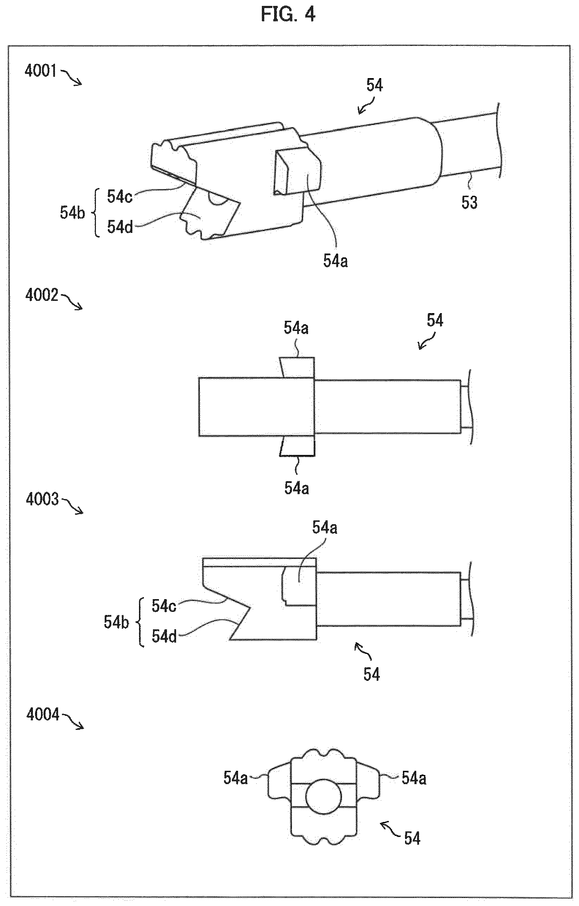

Fig. 4 illustrates a configuration of the lens contact member. 4001 of Fig. 4 is a perspective view of the lens contact member. 4002 of Fig. 4 is a top view of the lens contact member. 4003 of Fig. 4 is a side view of the lens contact member. 4004 of Fig. 4 is a front view of the lens contact member, as viewed from a front side thereof.
5001 and 5002 of Fig. 5 are cross-sectional views each illustrating behavior of a lens pressing part in a nozzle part.

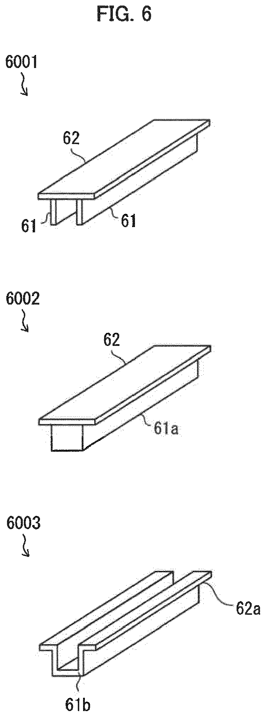

Fig. 6 is a perspective view illustrating examples of a configuration of a protruding part provided to the lens pressing part of the nozzle part. 6001 of Fig. 6 illustrates a configuration of a plate-like rib part illustrated in Fig. 5. 6002 of Fig. 6 illustrates an example of the configuration of the protruding part illustrated in 6001. 6003 of Fig. 6 illustrates another example of the configuration of the protruding part illustrated in 6001.

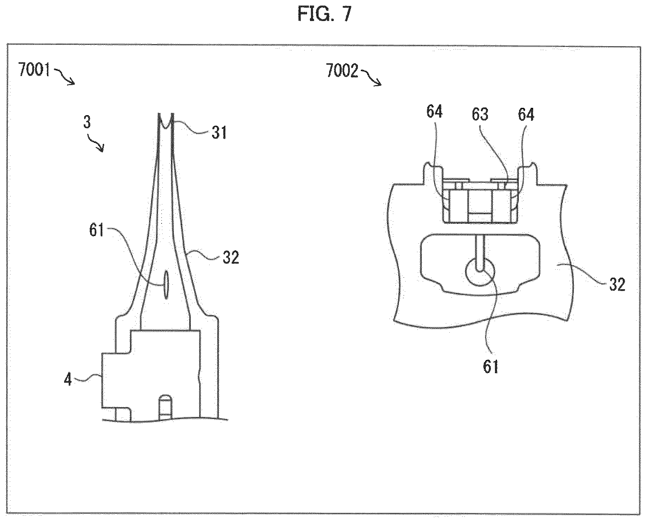

Fig. 7 illustrates the lens pressing part attached to the nozzle part. 7001 of Fig. 7 is a bottom view of the lens pressing part, as viewed from a lower side thereof. 7002 is a cross-sectional view illustrating a cross section of the lens pressing part which cross section is perpendicular to a forward-rearward direction.
8001 through 8005 of Fig. 8 are drawings each illustrating a cross-sectional shape of a tapered inner wall surface which defines a bore of the nozzle part.
9001 through 9005 of Fig. 9 are drawings each illustrating another cross-sectional shape of the tapered inner wall surface which defines the bore of the nozzle part.
10001 through 10005 of Fig. 10 are drawings each illustrating another cross-sectional shape of a ridge part provided on the tapered inner wall surface which defines the bore of the nozzle part.

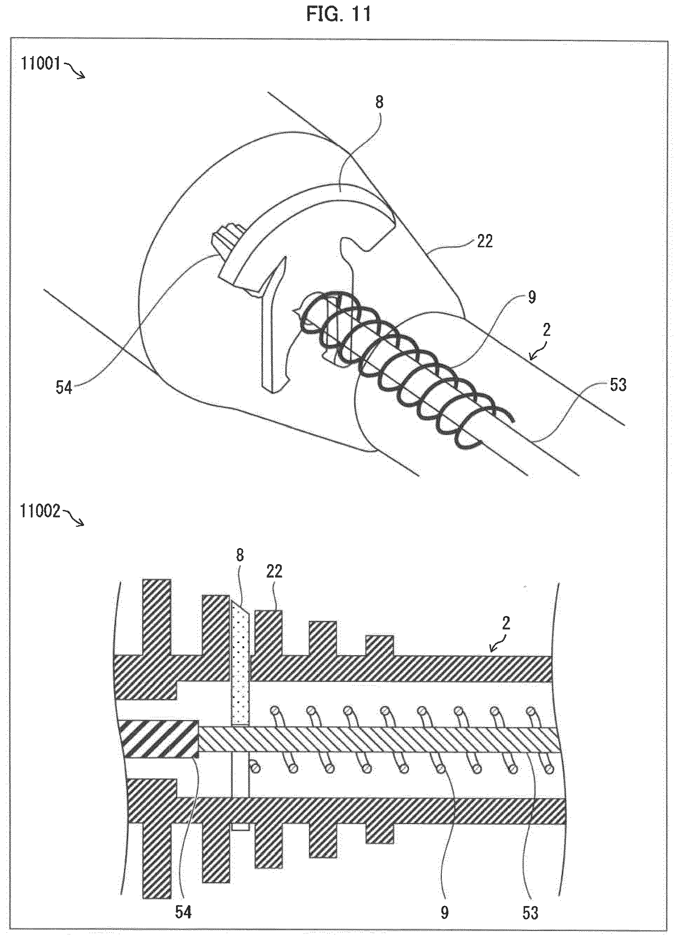

Fig. 11 illustrates a configuration of a centering member attached to a circular enlarged part. 10001 of Fig. 11 is a perspective view of the centering member. 11002 of Fig. 11 is a cross-sectional view of the centering member.
12001 of Fig. 12 is a front view illustrating the configuration of the centering member, as viewed from a front side thereof. 12002 of Fig. 12 is a cross-sectional view illustrating the configuration of the centering member attached to the circular enlarged part and illustrating a cross section thereof perpendicular to the forward-rearward direction.

### Description of Embodiments

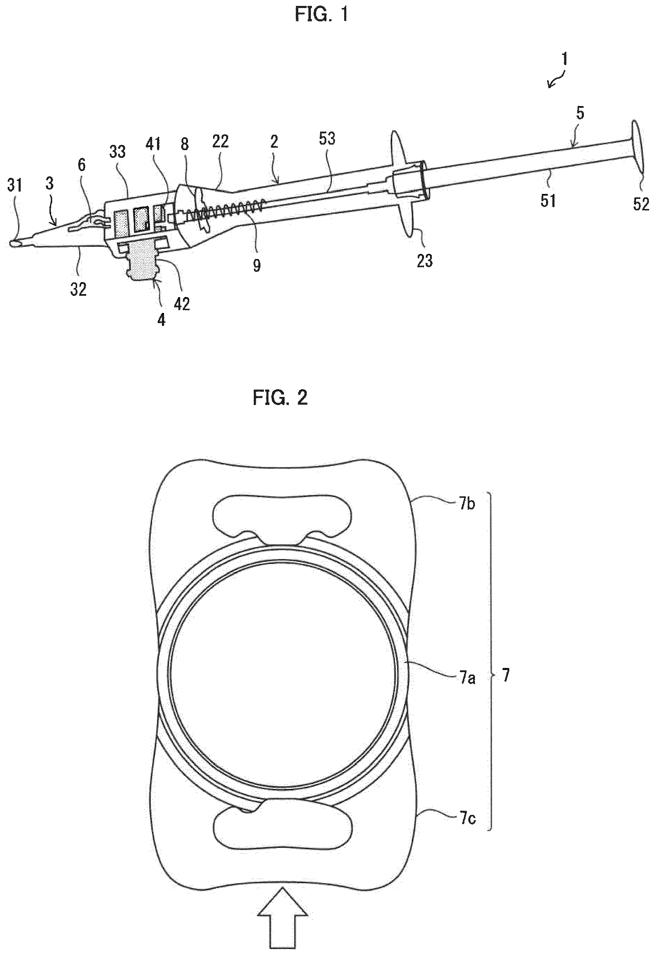

The following description will discuss an embodiment of the present invention in detail. Fig. 1 is a perspective view illustrating an entire configuration of an intraocular lens insertion implement 1 (hereinafter, referred to as an insertion implement 1). As illustrated in Fig. 1, the insertion implement 1 includes an implement body 2 which has the shape of a tube, a front-end chip 3, a lens holder 4 which is configured to hold an intraocular lens 7, a plunger 5, a lens pressing part 6, a centering member 8, and a spring 9. The front-end chip 3 has a nozzle part 32 which is connected to the implement body 2. The lens holder 4 is attachable to the implement body 2. The plunger 5 has the shape of a rod. The plunger 5 is configured to be inserted into the implement body 2. The lens pressing part 6 is provided to a rear portion of the nozzle part 32 of the front-end chip 3. The centering member 8 is a member for positioning of a central axis of the plunger 5. The centering member 8 is attached to the implement body 2. Note that the lens holder 4 may be attached to the front-end chip 3.

Note, here, that an axial direction of the plunger 5 is regarded as a forward-rearward direction. A thickness direction of the intraocular lens 7 which is held by the lens holder 4 extends is regarded as an upward-downward direction. A direction which is perpendicular to both of the forward-rearward direction and the upward-downward direction is regarded as a rightward-leftward direction. A side of the insertion implement 1 on which side the front-end chip 3 is located is regarded as a front side. A side of the insertion implement 1 which side is opposite from the front side is regarded as a rear side. A side of the front-end chip 3 on which side the lens pressing part 6 is located is regarded as an upper side. A side of the front-end chip 3 which side is opposite from the upper side is regarded as a lower side.

Fig. 2 is a top view illustrating a configuration of the intraocular lens 7 to be mounted on the lens holder 4 of the insertion implement 1. As illustrated in Fig. 2, the intraocular lens 7 includes a lens part 7a, a front support part 7b, and a rear support part 7c. The lens part 7a functions as a crystalline lens after the intraocular lens 7 is inserted into an eye. Each of the front support part 7b and the rear support part 7c supports the lens part 7a in the eye after the intraocular lens 7 is inserted into the eye. The front support part 7b and the rear support part 7c are provided so as to be symmetrical with respect to the lens part 7a. Each of the front support part 7b and the rear support part 7c has the shape of a flat plate. The front support part 7b is formed so as to cover a front side surface of the lens part 7a. The rear support part 7c is formed so as to cover a rear side surface of the lens part 7a. The intraocular lens 7 is a plate-type intraocular lens having a substantially rectangular outer shape. Such a rectangle is defined by the lens part 7a, the front support part 7b, and the rear support part 7c. Each of the lens part 7a, the front support part 7b, and the rear support part 7c is made of a flexible material, and is flexibly deformable. In the present embodiment, the intraocular lens 7 which is of a single piece type, that is, the intraocular lens 7 which is made up of the lens part 7a, the front support part 7b, and the rear support part 7c that are integrally molded will be explained as an example.

The implement body 2 is configured such that the front-end chip 3 is engaged with a front of the implement body 2. The implement body 2 has a circular enlarged part 22 on an outer surface of a front end thereof and a holding part 23 on an outer surface of a rear end thereof. The circular enlarged part 22 can be grasped by an operator. The holding part 23 has the shape of a flange, and is held by the operator putting his/her finger on the holding part 23. The implement body 2 is made of a resin having impact resistance, such as polycarbonate. The circular enlarged part 22 is constituted by, for example, a plurality of circular protrusions.

Note that each of the circular enlarged part 22 and the holding part 23 may have any shape, provided that each of the circular enlarged part 22 and the holding part 23 can carry out its required function. For example, the holding part 23 may be constituted by a protrusion or the like on which a finger can be put, instead of a flange.

The operator grasps the circular enlarged part 22 with one hand, while pushing the plunger 5 with the other hand. This causes the intraocular lens 7 to be released into a patient's eye in order of the front support part 7b, the lens part 7a, and the rear support part 7c. Providing the circular enlarged part 22 makes it easy for the operator to grasp the insertion implement 1. As a result, it is possible to enhance operability of the insertion implement 1.

The front-end chip 3 has (i) a release part 31 through which the intraocular lens 7 is released, (ii) a nozzle part 32 which has an inner diameter that gradually becomes smaller as the nozzle part 32 extends forward, and (iii) a rectangular part 33 which has a rectangular outer periphery with a side surface in which an opening is provided. By engaging the rectangular part 33 with the implement body 2, the front-end chip 3 is connected to the implement body 2. The lens pressing part 6 is attached to a vicinity of an entrance of the nozzle part 32 so that the lens pressing part 6 is located on an upper side of the nozzle part 32. Note that engagement of the front-end chip 3 with the implement body 2 may be achieved by any configuration. For example, the front-end chip 3 and the implement body 2 may be engaged with each other by a locking claw and a locking hole. The front-end chip 3 is made of a resin having chemical resistance and flexibility, such as polypropylene or polyamide.

The insertion implement 1 is configured such that the lens holder 4, which holds the intraocular lens 7, can be attached. The insertion implement 1 in accordance with the present embodiment may be in a form in which the lens holder 4 is separated or alternatively in a form in which the attached lens holder 4 is attached. When the operator uses the insertion implement 1, the operator inserts the lens holder 4 into the opening provided in the side surface of the rectangular part 33. It can be said that the rectangular part 33 is an installation part in which the lens holder 4 is installed. Note that the lens holder 4 may be inserted into the opening in the rectangular part 33 in advance before the insertion implement 1 is used. Alternatively, the lens holder 4 may be inserted into the opening in the rectangular part 33 when the insertion implement 1 is used.

The lens holder 4 has a housing part 41 and a tab 42. The housing part 41 defines a housing space in which the intraocular lens 7 is housed. The tab 42 is provided so as to protrude laterally from the housing part 41. An opening is provided on each of a front side and a rear side of the housing part 41. The space inside the housing part 41 is communicated with a space inside the nozzle part 32 via the opening provided on the rear side of the housing part 41. After the operator houses the intraocular lens 7 in the housing part 41, the operator pinches the tab 42, and inserts the lens holder 4 into the opening provided in the side surface of the rectangular part 33. This causes the intraocular lens 7 to be set in the insertion implement 1. The lens holder 4 is made of a resin having chemical resistance, such as polypropylene.

The plunger 5 includes a rear-side shaft part 51, a pressing part 52, and a middle shaft part 53. The rear-side shaft part 51 is located on the rearmost side of the plunger 5. The rear-side shaft part 51 is exposed outside, in a state where the plunger 5 is in an initial position in which the plunger 5 is inserted in the implement body 2. Rearward movement of the rear-side shaft part 51 is restricted by a stopper provided at the rear end of the implement body 2. The pressing part 52 is provided, in the shape of a flange, on a rear-side peripheral surface of the rear-side shaft part 51. The operator moves the plunger 5 forward by pushing the pressing part 52 toward the implement body 2.

The middle shaft part 53 is connected to a front of the rear-side shaft part 51, and has a diameter which is smaller than that of the rear-side shaft part 51. A lens contact member 54 is attached to a front end of the middle shaft part 53. The lens contact member 54 is configured to be brought into contact with the rear support part 7c of the intraocular lens 7.

The spring 9 is provided along an outer peripheral surface of the middle shaft part 53. As such, when the plunger 5 is pushed, the plunger 5 receives a reaction force from the spring 9. As a result, it is possible to prevent the intraocular lens 7 from briskly jumping out from the lens contact member 54 of the plunger 5. The spring 9 is not limited to any particular location, provided that the plunger 5 receives the reaction force from such an elastic member when the plunger 5 is pushed. For example, the spring 9 may be provided between the pressing part 52 of the plunger 5 and the rear end of implement body 2.

In a state where the plunger 5 is in the initial position, the operator pushes the plunger 5 forward from the initial position. This causes the plunger 5 to pass through the opening provided on the rear side of the housing part 41. The lens contact member 54 located at a front end of the plunger 5 is then brought into contact with the rear support part 7c of the intraocular lens 7. The plunger 5 is further moved forward to pass through the opening provided on the front side of the housing part 41. The plunger 5 is then moved into the front-end chip 3. In so doing, the intraocular lens 7 is folded in a recessed shape while the intraocular lens 7 is passing through the nozzle part 32 of the front-end chip 3. The intraocular lens 7 is then brought to the release part 31 in a state where the intraocular lens 7 is folded. The intraocular lens 7 brought to the release part 31 is inserted into an eye from a side of the release part 31 on which side the release part 31 is cut, in order of the front support part 7b, the lens part 7a, and the rear support part 7c.

By the way, an intraocular lens to be mounted on a conventional intraocular lens insertion implement disclosed in, for example, each of Patent Literatures 1 through 3 has a front support part and a rear support part which are formed so as to curvedly extend from respective side surfaces of a lens part. In this manner, each of the front support part and the rear support part has the shape of a loop. The intraocular lens 7, which is a plate-type intraocular lens as has been described above, is made of a flexible material, compared to the intraocular lens having the front support part and the rear support part each of which has the shape of a loop. Therefore, in a case where the intraocular lens 7, which is a plate-type intraocular lens, is inserted into an eye with use of an intraocular lens insertion implement which is configured such that a conventional intraocular lens is mounted thereon, the intraocular lens 7 may be broken. An object of the insertion implement 1 in accordance with the present embodiment is to make it possible to appropriately insert the intraocular lens 7, which is a plate-type intraocular lens and which is made of a flexible material, into an eye without causing a breakage of the intraocular lens 7.

### (Configuration of lens contact member 54)

Here, a position in the intraocular lens 7 with which position the plunger 5 is brought into contact may be misaligned with a position in the intraocular lens 7 with which position the plunger 5 should be brought into contact, due to, for example, an error in setting the intraocular lens 7 in the lens holder 4. This may cause the plunger 5 to go up onto or go under the intraocular lens 7. As has been described, the intraocular lens 7 is made of a flexible material. Therefore, the intraocular lens 7 may be damaged and broken by the plunger 5.

Furthermore, since the intraocular lens 7 is made of a flexible material, the intraocular lens 7 is easily deformed rearward from the position in the intraocular lens 7 with which position the plunger 5 is in contact, by being pushed by the plunger 5. In a case where the intraocular lens 7 as described above is pushed into the nozzle part 32, which has a tapered shape, and then folded, a force which acts forward is exerted on a portion of the intraocular lens 7 with which portion the plunger 5 is in contact. On the other hand, a force is exerted on a potion other than the portion in which the plunger 5 is in contact, due to friction between (i) the portion other than the portion in which the plunger 5 is in contact and (ii) a tapered surface of the nozzle part 32, such that the portion other than the portion in which the plunger 5 is in contact remains on the tapered surface. This consequently causes the intraocular lens 7 to pass through the nozzle part 32 in a state where the intraocular lens 7 is excessively elongated rearward from the position in the intraocular lens 7 with which position the plunger 5 is in contact. As a result, the intraocular lens 7 may be broken.

According to the insertion implement 1 in accordance with the present embodiment, it is possible to prevent such a breakage of the intraocular lens 7, by the lens contact member 54 provided to the plunger 5. 3001 of Fig. 3 is a cross-sectional view illustrating a state where the lens contact member 54 of the plunger 5 is brought to an opening 41a on the rear side of the housing part 41. 3002 of Fig. 3 is an enlarged view of the state illustrated in 3001 of Fig. 3. Fig. 4 illustrates a configuration of the lens contact member 54. 4001 of Fig. 4 is a perspective view of the lens contact member 54. 4002 of Fig. 4 is a top view of the lens contact member 54. 4003 of Fig. 4 is a side view of the lens contact member 54. 4004 of Fig. 4 is a front view of the lens contact member 54, as viewed from a front side.

According to the insertion implement 1 in accordance with the present embodiment, the lens contact member 54 of the plunger 5, which lens contact member 54 is brought into contact with the intraocular lens 7, is made of an elastically deformable material which is softer than a material of the middle shaft part 53 of the plunger 5 and which is elastically deformable. Examples of the material of the lens contact member 54 include silicone and silicone elastomer.

As illustrated in 3001 and 3002 of Fig. 3 and 4001 through 4004 of Fig. 4, the lens contact member 54 has protrusions 54a which protrude from respective side surfaces of the lens contact member 54 which side surfaces are located in the rightward-leftward direction. The lens contact member 54 further has an alligator-jaw structure 54b which has a notch. A dimension of this cutout is greater than a dimension of the intraocular lens 7 in the upward-downward direction (thickness direction). The alligator-jaw structure 54b is configured such that, at least in the housing part 41 of the lens holder 4, the intraocular lens 7 is sandwiched, in the upward-downward direction, by the alligator-jaw structure 54b. The alligator-jaw structure 54b has an upper inclined part 54c and a lower inclined part 54d which face each other in the upward-downward direction. The lower inclined part 54d is constituted by an inclined surface which is joined to a lower surface 54e of the lens contact member 54. In other words, a surface perpendicular to the lower surface 54e is not interposed between the inclined surface of the lower inclined part 54d and the lower surface 54e. Note that, as used herein, the structure "an inclined surface which is joined to a lower surface 54e of the lens contact member 54" also includes a structure such that, in processing a joint structure of the inclined surface of the lower inclined part 54d and the lower surface 54e, an R-surface or the like is formed at a joint between the inclined surface of the lower inclined surface 54d and the lower surface 54e, depending on accuracy of a processing condition and/or the like.

A dimension of the lens contact member 54 in the upward-downward direction is equal to or greater than that of the opening 41a in the upward-downward direction, which opening 41a is located on the rear side of the housing part 41, and that of an opening 41b in the upward-downward direction, which opening 41b is on the front side of the housing part 41. More specifically, the dimension of the lens contact member 54 in the upward-downward direction is equal to or greater than a distance between walls of the housing part 41 which face each other in the upward-downward direction. With this configuration, in a case where the lens contact member 54 is inserted into the housing part 41, the lens contact member 54 is elastically deformed so that the lens contact member 54 fits between the walls of the housing part 41 which face each other in the upward-downward direction. This elastic deformation causes the upper inclined part 54c and the lower inclined part 54d of the alligator-jaw structure 54b to elastically deform so that a distance between the upper inclined part 54c and the lower inclined part 54d becomes shorter, and ultimately causes the upper inclined part 54c and the lower inclined part 54d to come into contact with an upper surface and a lower surface, respectively, of the intraocular lens 7. As a result, the intraocular lens 7 housed in the housing part 41 is sandwiched, in the upward-downward direction, by the alligator-jaw structure 54b. Therefore, even in a case where an error occurs in setting the intraocular lens 7 in the lens holder 4, there is no misalignment of the position in the intraocular lens 7 with which position the plunger 5 is in contact. Therefore, according to the insertion implement 1 in accordance with the present embodiment, it is possible to prevent the plunger 5 from going up onto or going under the intraocular lens 7.

Note that the upper limit of the dimension of the lens contact member 54 in the upward-downward direction can be set, as appropriate, depending on a material of the lens contact member 54, dimensions of the alligator-jaw structure 54b, and the like, provided that movement of the lens contact member 54 is not interfered by contact between the lens contact member 54 and walls of the housing part 41, the nozzle part 32, and the release part 31.

Furthermore, the lower inclined part 54d is constituted by the inclined surface which is joined to the lower surface 54e of the lens contact member 54. Thus, in a case where the plunger 5 is moved forward in a state where the lens contact member 54 is in contact with the intraocular lens 7, the rear support part 7c of the intraocular lens 7 is easily moved onto the lower inclined part 54d, so that the intraocular lens 7 easily and smoothly fits in the alligator-jaw structure 54b. Therefore, it is possible to cause the intraocular lens 7 to be sandwiched more reliably by the alligator-jaw structure 54b.

Note that, in a case where (i) a surface perpendicular to the lower surface 54e is interposed between the inclined surface of the lower inclined part 54d and the lower surface 54e and (ii) the plunger 5 is moved forward in a state where the lens contact member 54 is in contact with the intraocular lens 7, it is highly likely that the intraocular lens 7 is locked by such a perpendicular surface. Accordingly, the rear support part 7c of the intraocular lens 7 is not easily moved onto the lower inclined part 54d, so that the intraocular lens 7 does not easily fit in the alligator-jaw structure 54b.

Moreover, although not shown, the dimension of the lens contact member 54 in the upward-downward direction is preferably equal to or greater than (i) a distance between walls which define a bore of the release part 31 of the front-end chip 3 and which face each other in the upward-downward direction and (ii) a distance between walls which define a bore of the nozzle part 32 of the front-end chip 3 and which face each other in the upward-downward direction. With this configuration, even while the plunger 5 is being further moved forward from the lens holder 4 and is being caused to pass through the nozzle part 32 and the release part 31, the intraocular lens 7 is sandwiched by the alligator-jaw structure 54b of the lens contact member 54. Therefore, misalignment of the position in the intraocular lens 7 with which position the plunger 5 is in contact hardly occurs. As a result, the intraocular lens 7 is stably folded in the nozzle part 32. Furthermore, since the rear support part 7c of the intraocular lens 7 is sandwiched by the alligator-jaw structure 54b also in the release part 31, it is expected that the intraocular lens 7 is prevented from being suddenly released from the release part 31.

Each of the protrusions 54a of the lens contact member 54 functions as a stopper which restricts rearward elongational deformation of the intraocular lens 7. The rearward elongational deformation of the intraocular lens 7 can be also referred to as elongational deformation of the intraocular lens 7 in a direction opposite to a direction in which the intraocular lens 7 is pushed out by the plunger 5.

In a case where the intraocular lens 7 is pushed into the nozzle part 32, which has a tapered shape, and is folded, a portion of the intraocular lens 7 which portion is deformed rearward from the position in the intraocular lens 7 with which position the plunger 5 is in contact is locked by the protrusions 54a, which are provided on the respective side surfaces of the lens contact member 54. Therefore, rearward deformation of the portion from the position in the intraocular lens 7 with which position the plunger 5 is in contact is restricted by the protrusions 54a, and thus it is possible to prevent the intraocular lens 7 from passing through the nozzle part 32 in a state where the intraocular lens 7 is excessively elongated. As such, according to the insertion implement 1 in accordance with the present embodiment, it is possible to prevent excessive rearward elongation of the intraocular lens 7 in the nozzle part 32, and thereby possible to prevent a breakage of the intraocular lens 7.

Note that a dimension of the lens contact member 54 in the rightward-leftward direction can be set, as appropriate, depending on the material of the lens contact member 54, dimensions of the protrusions 54a, and the like, provided that the movement of the lens contact member 54 is not interfered by contact between the lens contact member 54 and walls of the housing part 41, the nozzle part 32, and the release part 31.

### (Lens pressing part 6)

In order for the plate-type intraocular lens 7 to be released from the release part 31 without being broken by being pushed by the plunger 5, it is important that the intraocular lens 7 be axisymmetrically folded in the nozzle part 32 such that the upper surface of the intraocular lens 7 is located inside. The lens pressing part 6 has a function of assisting the intraocular lens 7 in being folded in such a manner in the nozzle part 32. 5001 and 5002 of Fig. 5 are cross-sectional views each illustrating behavior of the lens pressing part 6 in the nozzle part 32. Fig. 7 illustrates the lens pressing part 6 attached to the nozzle part 32. 7001 of Fig. 7 is a bottom view of the lens pressing part 6, as viewed from the lower side thereof. 7002 of Fig. 7 is a cross-sectional view illustrating a cross section of the lens pressing part 6 which cross section is perpendicular to the forward-rearward direction.

As illustrated in 5001 and (b) of Fig. 5 and 7001 and 7002 of Fig. 7, the lens pressing part 6 includes a plate-like rib part 61 (protruding part), a base part 62 which supports the plate-like rib part 61, a shaft part 63, and a leaf spring part 64. Note, here, that a rotational mechanism of the plate-like rib part 61 of the insertion implement 1 includes at least the shaft part 63 and the leaf spring part 64.

The plate-like rib part 61 is a ridge which protrudes toward the bore of the nozzle part 32 and extends in the forward-rearward direction (direction in which the intraocular lens 7 is pushed out by the plunger 5) throughout a predetermined dimension. A lower-side edge of the plate-like rib part 61 is formed so as to have a shape of a single curved line raised downward throughout a length of the plate-like rib part 61 in the forward-rearward direction. The plate-like rib part 61 is provided so that the plate-like rib part 61 is brought into contact with the plunger 5 and the lower-side edge is brought into contact with at least the lens part 7a of intraocular lens 7.

The shaft part 63 is provided so as to protrude in the rightward-leftward direction from the base part 62, and is inserted in a bearing opening provided to the nozzle part 32. The lens pressing part 6 is connected to the nozzle part 32 via the shaft part 63, and is rotatable on the shaft part 63, which extends in the rightward-leftward direction.

The leaf spring part 64 has a shape of a plate which is bent so as to be raised forward. The leaf spring part 64 is provided so as to be in contact with a predetermined planar surface of the nozzle part 32. The leaf spring part 64 is configured to bias the base part 62 so that the base part 62 is rotated toward the nozzle part 32. Therefore, in a case where the base part 62 is rotated on the shaft part 63 so as to be away from the nozzle part 32, a biasing force which causes the base part 62 to rotate toward the nozzle part 32 is exerted on the base part 62 by the leaf spring part 64.

In a case where the intraocular lens 7 is sent into the nozzle part 32 by being pushed by the plunger 5, a central portion of the lens part 7a of the intraocular lens 7 is brought into contact with the plate-like rib part 61. This brings the intraocular lens 7 into a state where the central portion of the intraocular lens 7 is positioned in a vicinity of an entrance of the nozzle part 32 by the plate-like rib part 61. In a case where the intraocular lens 7 is moved forward by being pushed by the plunger 5 in a state were the central portion of the intraocular lens 7 is positioned, the intraocular lens 7 is folded by the tapered surface of the nozzle part 32 without misalignment of the central portion. As a result, the intraocular lens 7 which has been caused to pass by the lens pressing part 6 and has been moved forward is folded axisymmetrically with respect to the central portion such that the upper surface of the intraocular lens 7 is located inside and the intraocular lens 7 is raised toward a bottom surface of the nozzle part 32.

The plunger 5 is pushed into the nozzle part 32 in a state where the lens contact member 54, which is located at the front end, is in contact with a rear end of the intraocular lens 7. Thus, in a case where the intraocular lens 7 is caused to pass by the lens pressing part 6 and is moved forward, the lens pressing part 6 is brought into one of the following states: (i) a lens surface of the intraocular lens 7 is in contact with the plate-like rib part 61 of the lens pressing part 6; (ii) the lens contact member 54 is in contact with the plate-like rib part 61 of the lens pressing part 6; or (iii) both of the lens surface of the intraocular lens 7 and the lens contact member 54 are in contact with the plate-like rib part 61 of the lens pressing part 6. In a case where the plunger 5 is further pushed in this state, the plate-like rib part 61 is rotated on the shaft part 63 so that the lower-side edge is away from the bottom surface of the nozzle part 32, due to a pressing force of the plunger 5. Therefore, advance of the plunger 5 is not interfered. Note that, in the present embodiment, even in a case where the lens surface of the intraocular lens 7 is in contact with the plate-like rib part 61 of the lens pressing part 6, the plate-like rib part 61 may be rotated due to contact between the lens pressing part 6 and the lens surface which is moved forward, when the plunger 5 is further pushed. That is, the plate-like rib part 61 may be rotated before the lens contact member 54 is brought into contact with the plate-like rib part 61.

Thereafter, in a case where the intraocular lens 7 is released from the release part 31 and then the plunger 5 is returned to a rear side of the lens pressing part 6, the biasing force of the leaf spring part 64 causes the plate-like rib part 61 to rotate toward the bottom surface of the nozzle part 32. The lens pressing part 6 is then returned to an initial position.

In this manner, according to the insertion implement 1, the intraocular lens 7 is axisymmetrically folded such that the upper side of the intraocular lens 7 is located inside, in a state where the central portion is positioned by the plate-like rib part 61. Thus, by being pushed by the plunger 5, the plate-type intraocular lens 7 is released from the release part 31 without being broken.

Furthermore, by the rotational mechanism including the shaft part 63 and the leaf spring part 64, the plate-like rib part 61 is rotated in the direction in which the intraocular lens 7 is pushed out by the plunger 5, in a case where the plunger 5 is brought into contact with the plate-like rib part 61. Therefore, even after the intraocular lens 7 has been caused to pass by the lens pressing part 6, the advance of the plunger 5 is not interfered by the lens pressing part 6. Accordingly, it is possible to smoothly push the plunger 5 while axisymmetrically folding the intraocular lens 7 in the nozzle part 32 such that the upper surface of the intraocular lens 7 is located inside.

In the present embodiment, a protruding part which extends in the forward-rearward direction (the direction in which the intraocular lens 7 is pushed out by the plunger 5) is not limited to the plate-like rib part 21 illustrated in Fig. 5, provided that the protruding part is configured to protrude toward the bore of the nozzle part 32 and position the central portion of the intraocular lens 7 as has been described above. Fig. 6 is a perspective view illustrating examples of the configuration of the protruding part. 6001 of Fig. 6 illustrates a configuration of the plate-like rib part 61 illustrated in Fig. 5. 6002 of Fig. 6 illustrates an example of the configuration of the protruding part illustrated in 6001 of Fig. 6. 6003 of Fig. 6 illustrates another example of the configuration of the protruding part illustrated in 6001 of Fig. 6.

As illustrated in 6001 of Fig. 6, according to the configuration illustrated in Fig. 5, two plate-like rib parts 61 are provided. The two plate-like rib parts 61 protrude toward the bore of the nozzle part 32 from the base part 62, and extend in the forward-rearward direction (direction in which the intraocular lens 7 is pushed out by the plunger 5) throughout a predetermined dimension such that the two plate-like rib parts 61 are parallel to each other. The two plate-like rib parts 61 are provided so as to be symmetrical with respect to a symmetry axis of the nozzle part 32.

Note that the number of protruding parts is not limited to two, unlike the plate-like rib parts 61 illustrated in 6001 of Fig. 6. As illustrated in 6002 of Fig. 6, a protruding part 61a may be a single ridge which protrudes toward the bore of the nozzle part 32 from the base part 62. The protruding part 61a is wide in the rightward-leftward direction to such a degree that the central portion of the intraocular lens 7 is positioned.

The protruding part 61a illustrated in 6002 of Fig. 6 is solid. However, the protruding part is not limited to a solid structure, and may have a hollow structure. 6003 of Fig. 6 illustrates an example of a configuration of a protruding part 61b having a hollow structure. As illustrated in 6003 of Fig. 6, the protruding part 61b is formed as a recessed groove which is recessed toward the bore of the nozzle part 32 from the base part 62a. The protruding part 61b extends in the forward-rearward direction (direction in which the intraocular lens 7 is pushed out by the plunger 5) throughout a predetermined dimension. The protruding part 61b is wide in the rightward-leftward direction to such a degree that the central portion of the intraocular lens 7 is positioned.

### (Shape of bore of nozzle part 32)

According to the insertion implement 1, the intraocular lens 7 is axisymmetrically folded in the nozzle part 32 such that the upper surface of the intraocular lens 7 is located inside. Thus, the insertion implement 1 is characterized by a shape of a tapered inner wall surface which defines the bore of the nozzle part 32. 8001 through 8005 of Fig. 8 are drawings each illustrating a cross-sectional shape of a tapered inner wall surface 35 which defines a bore 34 of the nozzle part 32. 9001 through 9005 of Fig. 9 are drawings each illustrating another cross-sectional shape of the tapered inner wall surface 35 which defines the bore 34 of the nozzle part 32. In each of Figs. 8 and 9, as cross-sectional shapes perpendicular to the forward-rearward direction, a cross-sectional shape at a I-I line, a cross-sectional shape at a II-II line, a cross-sectional shape at a III-III line, a cross-sectional shape at a IV-IV line, and a cross-sectional shape at a V-V line correspond to cross-sectional shapes 8001/9001 through 8005/9005, respectively, in order from the rear side to the front side.

As is clear from the cross-sectional shapes 8001 through 8005 illustrated in Fig. 8, the nozzle part 32 includes the bore 34, the tapered inner wall surface 35 which defines the bore 34, and ridge parts 36. Each of the ridge parts 36 is provided on an upper side of the tapered inner wall surface 35, and protrudes downward. Two ridge parts 36 are provided so that the symmetry axis of the nozzle part 32, which symmetry axis extends in the upward-downward direction, is sandwiched between the two ridge parts 36. The two ridge parts 36 are provided so as to be symmetrical with respect to the symmetry axis of the nozzle part 32. As is clear from the cross-sectional shapes 8004 and 8005 illustrated in Fig. 8, the ridge parts 36 are provided so as to avoid at least a front end portion of the nozzle part 32.

The intraocular lens 7 which has been moved into the nozzle part 32 by being pushed by the plunger 5 is folded by the lens pressing part 6, which is located in the vicinity of the entrance of the nozzle part 32, such that the upper surface of the intraocular lens 7 is located inside. The intraocular lens 7 is then further moved forward. Subsequently, the intraocular lens 7 which has been folded in such a manner is moved forward while being folded by an effect of the tapered inner wall surface 35. Note, here, that while the intraocular lens 7 which has been folded in such a manner is being moved on the tapered inner wall surface 35, an axis along which the intraocular lens 7 is advanced may be misaligned with an axis along which the intraocular lens 7 should be advanced. Misalignment of such an advance axis of the intraocular lens 7 causes the intraocular lens 7 to move while rotating along the tapered inner wall surface 35. As a result, there is a possibility that the intraocular lens 7 is not axisymmetrically folded such that the upper surface of the intraocular lens 7 is located inside.

In view of the above, according to the insertion implement 1, the ridge parts 36 are provided to the nozzle part 32. This allows such rotational movement of the intraocular lens 7 along the tapered inner wall surface 35 to be restricted. As is clear from the cross-sectional shapes 8002 and 8003 illustrated in Fig. 8, in a case where the intraocular lens 7 which has been folded such that the upper surface of the intraocular lens 7 is located inside is moved forward by the plunger 5, edges of the intraocular lens 7 in the rightward-leftward direction are locked by the ridge parts 36. Thus, movement of the intraocular lens 7 along the tapered inner wall surface 35 is restricted. Lock of the edges of the intraocular lens 7 by the ridge parts 36 is released in a vicinity of the front end portion of the nozzle part 32 in which front end portion the ridge parts 36 are not provided. As a result, the intraocular lens 7 is axisymmetrically folded in the nozzle part 32 such that the upper surface of the intraocular lens 7 is located inside. Note that it is preferable that the ridge parts 36 be provided on the rear side of a front-side end of the lens pressing part 6, from the viewpoint of restricting the rotational movement of the intraocular lens 7 along the tapered inner wall surface 35 immediately after the intraocular lens 7 is folded such that the upper surface of the intraocular lens 7 is located inside. Note also that, in the vicinity of the entrance of the nozzle part 32, it is not always necessary that the ridge parts 36 be provided on the rear side of the lens pressing part 6. This is because, in the vicinity of the entrance of the nozzle part 32, the edges of the intraocular lens 7 in the rightward-leftward direction do not reach positions of the ridge parts 36 of the tapered inner wall surface 35.

According to the configuration illustrated in Fig. 8, the cross-sectional shapes 8001 through 8005, which are perpendicular to the forward-rearward direction, of the tapered inner wall surface 35, which defines the bore 34 of the nozzle part 32, are shapes elongated in the upward-downward direction. However, the cross-sectional shapes of the tapered inner wall surface 35 are not limited to the cross-sectional shapes 8001 through 8005 illustrated in Fig. 8. The cross-sectional shapes of the tapered inner wall surface 35 can be set, as appropriate, in accordance with dimensions, behavior of folding, and the like of the intraocular lens 7. For example, the cross-sectional shapes of the tapered inner wall surface 35 may be the cross-sectional shapes 9001 through 9005, which are elongated in the rightward-leftward direction, as illustrated in Fig. 9.

According to the cross-sectional shapes 8001 through 8005 illustrated in Fig. 8, two ridge parts 36 are provided. However, according to the insertion implement 1 in accordance with the present embodiment, the number of ridge parts 36 is not particularly limited, provided that the ridge parts 36 are provided so as to be symmetrical with respect to the symmetry axis of the nozzle part 32.

For example, as is clear from cross-sectional shapes 10001 through 10005 illustrated in Fig. 10, the nozzle part 32 may be configured to include a single ridge part 36a. The ridge part 36a has a shape such that the ridge part 36a is symmetrical with respect to the symmetry axis of the nozzle part 32, and is provided so as to avoid at least the front end portion of the nozzle part 32. Further, the nozzle part 36a has a shape such that a width of the nozzle part 36a in the rightward-leftward direction becomes narrower as the nozzle part 36a extends toward the front end portion of the nozzle part 32. Even with the cross-sectional shapes 10001 through 10005 illustrated in Fig. 10, the intraocular lens 7 is axisymmetrically folded in the nozzle part 32 such that the upper surface of the intraocular lens 7 is located inside.

### (Centering member 8)

In order for the intraocular lens 7 to be axisymmetrically folded in the nozzle part 32 such that the upper surface of the intraocular lens 7 is located inside, it is important that, in the lens holder 4, the position in the intraocular lens 7 with which position the plunger 5 is in contact be not misaligned with the advance axis of the intraocular lens 7. The middle shaft part 53 of the plunger 5 is formed so as to be thinner than the rear-side shaft part 51. Therefore, in a case where a force is applied to the plunger 5, wobble occurs due to distortion of the middle shaft part 53. As a result, the position in the intraocular lens 7 with which position the plunger 5 is in contact may be misaligned with the advance axis of the intraocular lens 7. The centering member 8 is a member for restricting such wobble of the plunger 5.

Fig. 11 illustrates a configuration of the centering member 8 attached to the circular enlarged part 22. 11001 of Fig. 11 is a perspective view of the centering member 8. 11002 of Fig. 11 is a cross-sectional view of the centering member 8. 12001 of Fig. 12 is a front view illustrating the configuration of the centering member 8, as viewed from a front side thereof. 12002 of Fig. 12 is a cross-sectional view illustrating the configuration of the centering member 8 attached to the circular enlarged part 22 and illustrating a cross section thereof perpendicular to the forward-rearward direction.

As illustrated in 11001 of Fig. 11, the centering member 8 is provided in the circular enlarged part 22 of the implement body 2. A position of the centering member 8 in the implement body 2 is not limited to the circular enlarged part 22. The plunger 5 is attached so that the lens contact member 54 is located on a front side of the centering member 8. The spring 9 is provided on a rear side of the centering member 8. This configuration causes the lens contact member 54 of the plunger 5 to move only forward from the centering member 8 and not to move rearward from the centering member 8. For example, in a case where the circular enlarged part 22 is constituted by a plurality of circular protrusions, the centering member 8 is provided so as to be sandwiched between adjacent ones of the plurality of circular protrusions, as illustrated in 11002 of Fig. 11.

As illustrated in 12001 of Fig. 12, the centering member 8 has two lower locking claws 81, a plunger insertion part 82, and an upper locking piece 83. The plunger insertion part 82 is provided between the lower locking claws 81 and the upper locking piece 83. The middle shaft part 53 of the plunger 5 is inserted in the plunger insertion part 82. On an upper side of the circular enlarged part 22, a through hole is provided through which the plunger insertion part 82 can be caused to pass in the upward-downward direction. On a lower side of the circular enlarged part 22, two through holes are provided through which the respective lower locking claws 81 can be caused to pass.

The lower locking claws 81 are hooked on the respective two through holes from outside the circular enlarged part 22 through the respective two through holes, which are provided on the lower side of the circular enlarged part 22, so that the lower locking claws 81 are locked. The upper locking piece 83 is in contact with an edge of the through hole, which is provided on the upper side of the circular enlarged part 22 and through which the plunger insertion part 82 is caused to pass, from outside the circular enlarged part 22 so that the upper locking piece 83 is locked. By the lower locking claws 81 and the upper locking piece 83 being locked to the circular enlarged part 22 in this manner, the plunger insertion part 82 is aligned with an advance axis of the plunger 5. Therefore, even in a case where a force is applied to the plunger 5 by the plunger 5 being inserted into the plunger insertion part 82, wobble in the rightward-leftward direction and in the upward-downward direction is restricted. As a result, according to the insertion implement 1, the position in the intraocular lens 7 with which position the plunger 5 is in contact is not misaligned with the advance axis of the intraocular lens 7.

The centering member 8 may be formed separately from the implement body 2 or may be integrally formed with the implement body 2.

Aspects of the present invention can also be expressed as follows:
An intraocular lens insertion implement in accordance with a first aspect of the present invention is an intraocular lens insertion implement to which a lens holder configured to hold an intraocular lens is attachable, including: a plunger which is configured to be brought into contact with the intraocular lens held by the lens holder and which is configured to cause the intraocular lens to be pushed out; and a nozzle part from which the intraocular lens that has been pushed out by the plunger is released outside while the intraocular lens is being folded, the plunger including a front end part which is configured to be brought into contact with the intraocular lens and which is made of an elastically deformable material, the front end part including a stopper which is configured to restrict elongational deformation of the intraocular lens in a direction opposite to a direction in which the intraocular lens is pushed out by the plunger.

The intraocular lens insertion implement in accordance with a second aspect of the present invention is preferably arranged such that, in the first aspect, the stopper is a protrusion provided on a side surface of the front end part.

The intraocular lens insertion implement in accordance with a third aspect of the present invention is preferably arranged such that, in the first aspect or the second aspect, the front end part has an alligator-jaw structure which has a notch and which is configured such that the intraocular lens is sandwiched, in a thickness direction of the intraocular lens, by the alligator-jaw structure.

The intraocular lens insertion implement in accordance with a fourth aspect of the present invention is preferably arranged such that, in the third aspect, the alligator-jaw structure has a lower inclined part and an upper inclined part which face each other in the thickness direction of the intraocular lens; and the lower inclined part is constituted by an inclined surface which is joined to a lower surface of the front end part.

An intraocular lens insertion implement in accordance with a fifth aspect of the present invention is an intraocular lens insertion implement to which a lens holder configured to hold an intraocular lens is attachable, including: a plunger which is configured to be brought into contact with the intraocular lens held by the lens holder and which is configured to cause the intraocular lens to be pushed out; and a nozzle part from which the intraocular lens that has been pushed out by the plunger is released outside while the intraocular lens is being folded, the nozzle part including: a plate-like rib part which is provided so as to be located in the middle of the nozzle part and which extends in a direction in which the intraocular lens is pushed out by the plunger; and a rotational mechanism which is configured to rotate the plate-like rib part in the direction in which the intraocular lens is pushed out by the plunger, by contact of a lens surface of the intraocular lens and/or the plunger with the plate-like rib part.

An intraocular lens insertion implement in accordance with a sixth aspect of the present invention is an intraocular lens insertion implement to which a lens holder configured to hold an intraocular lens is attachable, including: a plunger which is configured to be brought into contact with the intraocular lens held by the lens holder and which is configured to cause the intraocular lens to be pushed out; and a nozzle part from which the intraocular lens that has been pushed out by the plunger is released outside while the intraocular lens is being folded, the nozzle part including: a protruding part which is provided so as to be located in the middle of the nozzle part and which protrudes toward a bore of the nozzle part and extends in a direction in which the intraocular lens is pushed out by the plunger; and a rotational mechanism which is configured to rotate the protruding part in the direction in which the intraocular lens is pushed out by the plunger, by (i) contact of a lens surface of the intraocular lens with the protruding part, (ii) contact of the plunger with the protruding part, or (iii) contact of both of the lens surface of the intraocular lens and the plunger with the protruding part.

The intraocular lens insertion implement in accordance with a seventh aspect of the present invention is preferably arranged such that, in the sixth aspect, the protruding part is a plate-like rib part which protrudes toward the bore of the nozzle part and extends in the direction in which the intraocular lens is pushed out by the plunger.

An intraocular lens insertion implement in accordance with an eighth aspect of the present invention is an intraocular lens insertion implement to which a lens holder configured to hold an intraocular lens is attachable, including: a plunger which is configured to be brought into contact with the intraocular lens held by the lens holder and which is configured to cause the intraocular lens to be pushed out; and a nozzle part from which the intraocular lens that has been pushed out by the plunger is released outside while the intraocular lens is being folded, the nozzle part having a tapered inner wall surface which is configured such that a distance between wall surfaces that face each other becomes shorter as the wall surfaces extend in a direction in which the intraocular lens is pushed out by the plunger, the tapered inner wall surface being provided with a ridge part which extends in the direction in which the intraocular lens is pushed out by the plunger and which is configured to restrict rotational movement of the intraocular lens along the tapered inner wall surface.

The intraocular lens insertion implement in accordance with a ninth aspect of the present invention is preferably arranged such that, in the eighth aspect, the ridge part is provided so as to be symmetrical with respect to an axis of the nozzle part and is provided so as to avoid at least a front end portion of the nozzle part.

The intraocular lens insertion implement in accordance with a tenth aspect of the present invention is preferably arranged such that, in the ninth aspect, the ridge part includes at least two ridge parts; and the at least two ridge parts are provided so as to be symmetrical with respect to the axis of the nozzle part.

An intraocular lens insertion implement in accordance with a eleventh aspect of the present invention is an intraocular lens insertion implement which includes a body having a shape of a tube and to which a lens holder configured to hold an intraocular lens is attachable on an end surface side of the body, including: a plunger which is configured to (i) be inserted into the body so as to be capable of entering and leaving the body, (ii) be brought into contact with the intraocular lens held by the lens holder, and (iii) cause the intraocular lens to be pushed out; and a nozzle part from which the intraocular lens that has been pushed out by the plunger is released while the intraocular lens is being folded, the body being provided with a centering member which is configured to center the plunger.

The intraocular lens insertion implement in accordance with a twelfth aspect of the present invention is arranged such that, in any one of the fifth aspect through the seventh aspect, the rotational mechanism includes: a shaft part which is provided so as to protrude in a rightward-leftward direction from a base part that supports the plate-like rib part; and a leaf spring part which is configured to bias the base part so that the base part rotates toward the nozzle part.

The present invention is not limited to the embodiments, but can be altered by a skilled person in the art within the scope of the claims. The present invention also encompasses, in its technical scope, any embodiment derived by combining technical means disclosed in differing embodiments.

### Reference Signs List

1 Insertion implement (intraocular lens insertion implement)
2 Implement body
3 Front-end chip
4 Lens holder
5 Plunger
7 Intraocular lens
8 Centering member
22 Circular enlarged part
32 Nozzle part
35 Tapered inner wall surface
36, 36a Ridge part
41 Housing part
41a, 41b Opening
54 Lens contact member
54a Protrusion (stopper)
54b Alligator-jaw structure
54c Upper inclined part
54d Lower inclined part
54e Lower surface
61 Plate-like rib part (protruding part)
62 Base part
63 Shaft part
64 Leaf spring part
81 Lower locking claw
82 Plunger insertion part
83 Upper locking piece

## Claims

1. An intraocular lens insertion implement to which a lens holder configured to hold an intraocular lens is attachable, comprising:
a plunger which is configured to be brought into contact with the intraocular lens held by the lens holder and which is configured to cause the intraocular lens to be pushed out; and
a nozzle part from which the intraocular lens that has been pushed out by the plunger is released outside while the intraocular lens is being folded,
the plunger including a front end part which is configured to be brought into contact with the intraocular lens and which is made of an elastically deformable material,
the front end part including a stopper which is configured to restrict elongational deformation of the intraocular lens in a direction opposite to a direction in which the intraocular lens is pushed out by the plunger.

2. The intraocular lens insertion implement as set forth in claim 1, wherein the stopper is a protrusion provided on a side surface of the front end part.

3. The intraocular lens insertion implement as set forth in claim 1 or 2, wherein the front end part has an alligator-jaw structure which has a notch and which is configured such that the intraocular lens is sandwiched, in a thickness direction of the intraocular lens, by the alligator-jaw structure.

4. The intraocular lens insertion implement as set forth in claim 3, wherein:
the alligator-jaw structure has a lower inclined part and an upper inclined part which face each other in the thickness direction of the intraocular lens; and
the lower inclined part is constituted by an inclined surface which is joined to a lower surface of the front end part.

5. An intraocular lens insertion implement to which a lens holder configured to hold an intraocular lens is attachable, comprising:
a plunger which is configured to be brought into contact with the intraocular lens held by the lens holder and which is configured to cause the intraocular lens to be pushed out; and
a nozzle part from which the intraocular lens that has been pushed out by the plunger is released outside while the intraocular lens is being folded,
the nozzle part including:
a plate-like rib part which is provided so as to be located in the middle of the nozzle part and which extends in a direction in which the intraocular lens is pushed out by the plunger; and
a rotational mechanism which is configured to rotate the plate-like rib part in the direction in which the intraocular lens is pushed out by the plunger, by contact of a lens surface of the intraocular lens and/or the plunger with the plate-like rib part.

6. An intraocular lens insertion implement to which a lens holder configured to hold an intraocular lens is attachable, comprising:
a plunger which is configured to be brought into contact with the intraocular lens held by the lens holder and which is configured to cause the intraocular lens to be pushed out; and
a nozzle part from which the intraocular lens that has been pushed out by the plunger is released outside while the intraocular lens is being folded,
the nozzle part including:
a protruding part which is provided so as to be located in the middle of the nozzle part and which protrudes toward a bore of the nozzle part and extends in a direction in which the intraocular lens is pushed out by the plunger; and
a rotational mechanism which is configured to rotate the protruding part in the direction in which the intraocular lens is pushed out by the plunger, by (i) contact of a lens surface of the intraocular lens with the protruding part, (ii) contact of the plunger with the protruding part, or (iii) contact of both of the lens surface of the intraocular lens and the plunger with the protruding part.

7. The intraocular lens insertion implement as set forth in claim 6, wherein the protruding part is a plate-like rib part which protrudes toward the bore of the nozzle part and extends in the direction in which the intraocular lens is pushed out by the plunger.

8. An intraocular lens insertion implement to which a lens holder configured to hold an intraocular lens is attachable, comprising:
a plunger which is configured to be brought into contact with the intraocular lens held by the lens holder and which is configured to cause the intraocular lens to be pushed out; and
a nozzle part from which the intraocular lens that has been pushed out by the plunger is released outside while the intraocular lens is being folded,
the nozzle part having a tapered inner wall surface which is configured such that a distance between wall surfaces that face each other becomes shorter as the wall surfaces extend in a direction in which the intraocular lens is pushed out by the plunger,
the tapered inner wall surface being provided with a ridge part which extends in the direction in which the intraocular lens is pushed out by the plunger and which is configured to restrict rotational movement of the intraocular lens along the tapered inner wall surface.

9. The intraocular lens insertion implement as set forth in claim 8, wherein the ridge part is provided so as to be symmetrical with respect to an axis of the nozzle part and is provided so as to avoid at least a front end portion of the nozzle part.

10. The intraocular lens insertion implement as set forth in claim 9, wherein:
the ridge part includes at least two ridge parts; and
the at least two ridge parts are provided so as to be symmetrical with respect to the axis of the nozzle part.

11. An intraocular lens insertion implement which includes a body having a shape of a tube and to which a lens holder configured to hold an intraocular lens is attachable on an end surface side of the body, comprising:
a plunger which is configured to (i) be inserted into the body so as to be capable of entering and leaving the body, (ii) be brought into contact with the intraocular lens held by the lens holder, and (iii) cause the intraocular lens to be pushed out; and
a nozzle part from which the intraocular lens that has been pushed out by the plunger is released while the intraocular lens is being folded,
the body being provided with a centering member which is configured to center the plunger.
